# EUROPEAN PATENT APPLICATION

(11) **EP 1 939 350 A1**
(43) Date of publication of application: **02.07.2008**
(21) Application number: 06077344.7
(22) Date of filing: 29.12.2006
(51) Int. Cl.: D06M 10/08, D06M 16/00, D06M 10/10, D06M 14/18

(54) **Substrate with antimicrobial coating**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Alkema, Duurt Pieter Willem, 2288 GJ Rijswijk (NL); Simor, Marcel, 2288 GJ Rijswijk (NL); Noort, Daniel, 2288 GJ Rijswijk (NL); Tromp, Maarten Cornelis, 2288 GJ Rijswijk (NL); van Hooft, Petrus Albertus Venantia, 2288 GJ Rijswijk (NL); Fiala, Ales, 2288 GJ Rijswijk (NL)
(74) Representative: van Loon, C.J.J.

(57) **Abstract**

The invention is directed to a process for preparing a substrate with an antimicrobial coating, and to a substrate with an antimicrobial coating. More, in particular the invention relates to a process in which an antimicrobial coating is reacted on the surface of a substrate using a plasma treatment.
It was found that the process of the invention hardly affects the physical properties of the substrate, is environmentally friendly, uses high molecular weight non-toxic active compounds, and have improved antimicrobial activity compared to conventional wet processing techniques.

## Description

The invention is directed to a process for preparing a substrate with an antimicrobial coating, to the use of specific antimicrobial compounds, and to a substrate with an antimicrobial coating.

For a wide range of applications it is desirable to provide substrates with antimicrobial properties. A typical way of providing antimicrobial properties to a substrate, such as textile, is by treating the substrate with an antimicrobial agent. In the art, the antimicrobial agent is typically applied from solution by wet processing techniques such as sol-gel, soaking, spraying, dipping, fluid-flow, padding, printing, spin coating, and dip coating. Application of antimicrobial coatings using wet processing techniques are for instance known from US-B-6 368 361, EP-A-0 908 553, WO-A-2004/050132, US-A-2003/0 056 297, US-A-2003/0 106 162, and US-A-2003/0 088 923. These wet processing techniques have the disadvantage that they are relatively environmentally unfriendly, since large amounts of chemicals and solvents are required and high energy costs are involved due to heating and drying steps. Furthermore, the physical properties of the substrate may be disadvantageously affected by the applied coating. In addition, some wet deposition processes are difficult to scale-up due to the complicated multi-step processing.

Object of the present invention is to overcome at least one of the above-mentioned disadvantages and to enhance performance of antimicrobial coatings by plasma treatment. This object has been met by the process of the invention according to which an antimicrobial coating is prepared on the surface of a substrate using a plasma treatment. Accordingly, the invention is directed to a process for preparing a substrate with an antimicrobial coating comprising:
- providing a substrate;
- subjecting a surface of the substrate to a plasma environment; and
- reacting an antimicrobial active compound on the surface of the substrate.

The inventors found that the physical properties of the substrate, such as weight, strength, feel, and breathability are hardly affected by the process of the invention. The plasma treatment is more environmentally friendly than the traditional wet processing. Furthermore, the process of the invention can be advantageously carried out at atmospheric pressure.

Surprisingly the plasma treatment does not necessarily cause the antimicrobial active compound to lose its activity. In addition, it was found that the antimicrobial coatings obtained by the plasma treatment of the invention had unexpected improved properties over antimicrobial coatings obtained by conventional wet processing techniques.

The use of a plasma environment for depositing a polymer layer on a substrate material is for example known from EP-A-1 090 178, US-B-6 551 950 and WO-A-03/084681 that describe a method of coating a surface with oil and/or water repellent polymer layers. Suitably, low power pulsed plasma polymerisation is used in order to produce well-adhered coatings which exhibit excellent water and oil repellency but not antimicrobial functionality.

The use of a plasma environment for depositing a polymer layer on a substrate material is also known from the non-prepublished international patent application PCT/NL2006/000344. However, this document is directed to nanoparticle containing polymer coatings and does not disclose a reactive antimicrobial active compound.

A way to achieve antimicrobial and multifunctional textiles is disclosed in WO-A-02/079563 and US-A-2003/0056297. The multifunctional textile composition comprises a textile having an antimicrobial functionality and a chemical agent attached thereto to impart an additional functionality, which includes waterproof finishing, soil repellent finishing, fire resistance finishing, wrinkle free finishing, anti-UV finishing, and antistatic finishing. Main limitation of the inventions is that the antimicrobial functionality is a property of the textile composition, which limits the variety of substrate materials. The multifunctional textile of the present invention may be prepared by deposition of a coating on virtually any substrate material.

The term "antimicrobial" as used in this application is meant to refer to the ability to destroy at least some types of micro-organisms, inhibit development, growth or reproduction of at least some types of micro-organisms or inhibit their pathogenic action.

The term "plasma" as used in this application is meant to refer to a partially ionised gas that represents a chemically active environment, which consists of active species such as electrons, ions, radicals, metastables and photons.

The term "plasma polymerisation" as used in this application is meant to refer to the procedure, in which polymerisable materials, stimulated through a plasma, condense as polymers.

The term "surface" as used in this application is meant to refer to outer surfaces of a substrate, but also to outer surfaces of fibres in a substrate, inner surfaces of porous fibres in a substrate, and inner surfaces of pores in a substrate.

The term "textile" as used in this application is meant to refer to a thin, flexible material made of any combination of cloth, fibre, or polymer.

The term "cloth" as used in this application is meant to refer to a thin, flexible material made from yarns.

The term "yarn" as used in this application is meant to refer to a continuous strand of fibres.

The term "fibre" as used in this application is meant to refer to a unit of matter, either natural, such as cotton, synthetic, such as polyester, or a combination thereof, which forms the basic element of, for example, fabrics, and textile structures. A fibre itself may have a porous structure with voids.

In accordance with the invention an antimicrobial coating is applied to a substrate. Thereafter an antimicrobial active compound is reacted on the surface of the substrate, before, while or after said surface is subjected to a plasma treatment.

Any type of plasma source may be used. Typical plasma sources include corona discharge, atmospheric pressure glow discharge, microwave discharge, volume filamentary dielectric barrier discharge, volume glow dielectric barrier discharge, plasma jet, micro-hollow cathode discharge, surface dielectric barrier discharge, and coplanar surface dielectric barrier discharge. Any power source, such as continuous and pulsed, may be used to create plasma. It is preferred that the power source is a pulsed power source, since this allows a better control over plasma chemistry.

Particularly preferred plasma sources are dielectric barrier discharges (DBDs). In the case of surface DBD, the electrode structure of the plasma source comprises two electrodes arranged on opposite sides of a dielectric. Coplanar surface DBD is a special case of surface DBD in which metallic parts of electrodes are embedded in a dielectric and are not in direct contact with plasma, thus resulting in a longer lifetime of the electrodes.

Surface DBD plasma sources can generate a stable atmospheric pressure plasma not only in helium but, in nearly any gas mixture. Surface DBD plasma sources are very suitable for the treatment of surfaces and for the treatment of textile materials in particular. The reason for this is that in surface DBD plasma sources the plasma channels are parallel with a substrate surface and plasma is thus in a good contact with the surface. A further advantage of surface DBD plasma sources is that all surfaces, not only outer surfaces but also inner surfaces, are treated by plasma.

As an example, in the case of a fibrous substrate, such as various textiles, material is deposited around individual fibres that are on the surface but also in the bulk of the substrate. The consequence is that an enormous surface area may be covered. Further, voids between fibres are not filled and the fabric keeps its breathability.

A plasma jet and microwave plasma sources are particularly suitable for treating three-dimensional and/or non-flat substrates.

In one embodiment, the plasma treatment comprises a so-called plasma assisted grafting technique. This is a two-step process comprising plasma activation of the surface of the substrate followed by the exposure of the surface to a liquid or gaseous precursor, which may also be a gasified liquid or an atomised (sublimated) solid. Plasma activation of the substrate surface comprises hydrogen abstraction, radical formation and introduction of new functional groups from the plasma environment. New functional groups may also be introduced on the substrate surface from the surrounding air after plasma activation. The plasma activation results in a reactive activated surface.

In the second step of the plasma assisted grafting technique, a antimicrobial active compound is reacted on the surface after the surface is activated by the plasma. The antimicrobial active compound can undergo a conventional free radical reaction onto the activated surface of the substrate. The result is a very thin antimicrobial coating on the surface of the substrate.

The antimicrobial active compound may be provided on the surface in liquid or gaseous form. It is also possible to provide the antimicrobial active compound by a conventional wet processing technique, i.e. from solution.

It was surprisingly found that antimicrobial coatings prepared by the plasma assisted grafting technique have a better antimicrobial activity than antimicrobial coatings prepared by conventional wet processing. For example antimicrobial and hydrophilic coatings were deposited by dipping preceded by a plasma pre-treatment. Plasma activation in nitrogen plasma with and without water vapour enhanced the antimicrobial performance of the coating by more than one order of magnitude.

According to another embodiment, which may optionally be combined with the first embodiment, the antimicrobial active compound is provided on the surface of the substrate in the plasma environment. The reactive antimicrobial active compound is then plasma polymerised on the surface of the substrate, resulting in a very thin antimicrobial coating on the surface of the substrate, for example with a thickness from several nanometers to hundreds of nanometers.

Polymers formed by the process of plasma polymerisation can have different chemical and physical properties from those formed by conventional polymerisation. Plasma polymerised films can be highly cross-linked and can, therefore, have many appealing characteristics such as thermal stability, chemical inertness, mechanical toughness and negligible ageing. Also the washing-off characteristics can be enhanced.

In contrast to the conventional wet processing techniques, the plasma polymerisation process of this embodiment of the invention advantageously does not require liquid baths comprising toxic or harmful chemicals. Further, no heating, drying and/or curing activities are needed thereby reducing operational costs.

The plasma polymerisation process has also been identified as plasma assisted vapour deposition, plasma enhanced vapour deposition, plasma (assisted) chemical vapour deposition and plasma enhanced chemical vapour deposition.

In the third embodiment, which may optionally be combined with previous embodiments, the plasma treatment comprises a so-called plasma induced polymerisation technique. This is a two-step process in which an antimicrobial active compound is provided on the surface of substrate followed by the exposure of the said surface to plasma environment.

The plasma induced polymerisation has also been identified as plasma induced grafting.

Plasma conditions can be found such that antimicrobial coatings prepared by plasma polymerisation and plasma induced polymerisation have a better antimicrobial activity than antimicrobial coatings prepared by conventional wet processing. A plasma activation of the substrate prior to plasma polymerising or plasma induced polymerising the antimicrobial active compound further enhances the antimicrobial performance.

It is advantageous that the plasma treatment is carried out under substantially atmospheric pressure. Cost for providing low pressure circumstances, such as for example required in the case of metal sputtering or metal evaporation techniques, can thus be avoided.

The resulting antimicrobial coating on the surface of the substrate is very thin, preferably less then 500 nm, more preferably 50-200 nm, most preferably 10-100 nm. Such thin coatings impart the desired properties to the substrate without significantly affecting the original characteristics of the original substrate, such as for instance breathability, feel and softness.

A major advantage of the present invention is that further functionalities can be introduced on the surface of the substrate in a minimum of processing steps. This may be achieved for instance by using multifunctional compounds. It is also possible to use different functional chemicals simultaneously with the reactive antimicrobial active compound or separately before or after deposition of the reactive antimicrobial compound in the process according to the invention in order to include additional functionalities.

Additional substrate functionalities that may be introduced or influenced are for instance hydrophobicity, dirt and soil repellence, oleophobicity, hydrophilicity, anti-static, anti-UV and flame retardation and resistance properties.

Nanoparticle additives, such as zinc oxide (ZnO), titanium dioxide (TiO₂), or silver (Ag) nanoparticles may also be included in the polymer coating. These additives can provide for example UV-protection or self-cleaning functionality to the substrate or further improve the antimicrobial functionality of the substrate. The nanoparticle additives may be any structure with a size in the nanometer and micrometer range. Those structures include for example nanoparticles, nanofibres and/or nanotubes or a combination of them, such as nanotubes with attached and/or embedded nanoparticles and/or nanoparticles with one or more elements, such as a drug, dye and/or fragrance encapsulated. Further the nanoparticle additives can comprise either one type of nanoparticle additive or a mixture of nanoparticle additives. Preferably, the nanoparticle additive comprises a metal and/or a metal oxide in order to result in specific enhanced features of the substrate. Instead of using metals and/or metal oxides, other organic, inorganic nanoparticle additives or inorganic-organic composites could be used. Nanoparticles are preferably deposited, if allowed by the structure of the substrate, on the level of individual fibres and on inner surfaces of pores of a substrate thus covering an enormous surface area. Nanoparticles can be deposited for example by plasma polymerisation, plasma assisted grafting or plasma induced grafting (polymerization).

The above-mentioned embodiments of the invention may be further combined with conventional wet processing techniques.

Irrespective of the way by which the antimicrobial coatings are applied according to the present invention, they are strongly bound to the surface of the substrate and maintain their antimicrobial activity.

The substrate can be any substantially flat material, such as non-woven or woven textile, a nanofibre non-woven, felt, yarn, fibre, paper, foil, membranes, leather, polymers, wood and/or ceramics. Preferably, the substrate comprises yarns and fibres. Particularly preferred substrates are textiles, yarns and fibres.

Preferably, the antimicrobial active compound comprises a reactive group. Said reactive group can be a linker attached to a known or new antimicrobial compound or can be part of a known or new antimicrobial compound.

The antimicrobial active compound may comprise a quaternary ammonium moiety. Preferably the antimicrobial active compound comprises an optionally substituted aromatic heterocycle with at least one nitrogen atom. Examples of such aromatic heterocycles are five or six membered rings with at least one nitrogen atom, such as imidazoles, triazoles, tetrazoles, pyridines, pyrroles, pyrimidines, pyridazine, pyrazine, purine, or (benzo-fused) derivatives thereof. The aromatic heterocycle may be substituted with one or more functional groups, such as a hydroxyl or an amino group. A preferred aromatic heterocycle is an imidazole or derivative thereof.

The reactive group is preferably a (meth)acrylate or derivative thereof, a hydroxyl or an amino group.

The reactive group may be bound through an isocyanate, glycidyl, an alcohol, an epoxide, a vinyl or an allyl moiety. It is also possible that the reactive group is linked by a spacer. The spacer may be a flexible carbon chain of typically 1-12 carbon atoms, preferably 2-8 carbon atoms.

Preferably, the antimicrobial active compound is a quaternary ammonium compound according to general formula (I) 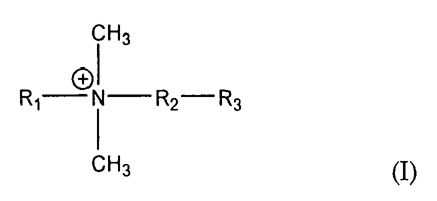 wherein R₁ is a C₃₋₁₂ alkyl group, preferably a C₅₋₉ alkyl group, more preferably a C₇ or C₈ alkyl group,
wherein R₂ is an optionally substituted C₁₋₆ chain, preferably a C₂₋₄ chain, and
wherein R₃ is chosen from the group consisting of an acrylate, a diisocyanate, a diol, an ethylene, and a styrene.
The counter ion can be a halogen ion, preferably chloride or bromide.

Another preferred antimicrobial active compound is a quaternary imidazole compound according to general formula (II) 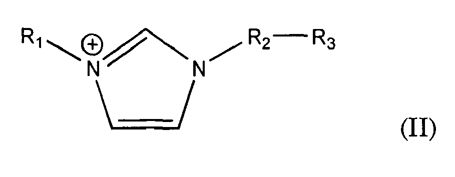 wherein R₁, R₂ and R₃ have the above identified meanings.

In case R₃ is an acrylate, the quaternary ammonium compounds and the imidazole compound may be provided on the surface of the substrate together with acrylic acid or (meth)acrylate monomers, such as butylmethacrylate, to provide a copolymer. The molar ratio between the antimicrobial agent and the acrylate monomer is preferably from 1:10 to 1:2, more preferably from 1:9 to 1:3.

It was found that the above-mentioned quaternary ammonium and quaternary imidazole compounds show a significant better antimicrobial activity than the commercially available antimicrobial agent DOW 5700 (octadecyldimethyl(3-trimethoxysilylpropyl)ammoniumchloride), also known as Aegis™, which has the following structure. 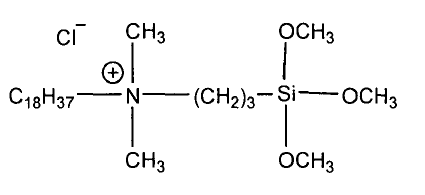 The above-mentioned quaternary ammonium and quaternary imidazole compounds also have a significant better antimicrobial activity than the commercially available silver-based antimicrobial agent Silpure, and compounds according to general formula (I) and (II) wherein the R₁ alkyl group has more than twelve carbon atoms.

It was further found that antimicrobial active compounds comprising a spacer located at R₂ based on poly(propylene oxide) or a polyethylene oxide or polytetramethyleneoxide showed enhanced antimicrobial behaviour, probably due to better adsorption of proteins to the surface.

Some examples of preferred antimicrobial compounds are shown in Table I.

**Table 1 Examples of preferred quaternary ammonium compounds.**

| | |
|---|---|
| 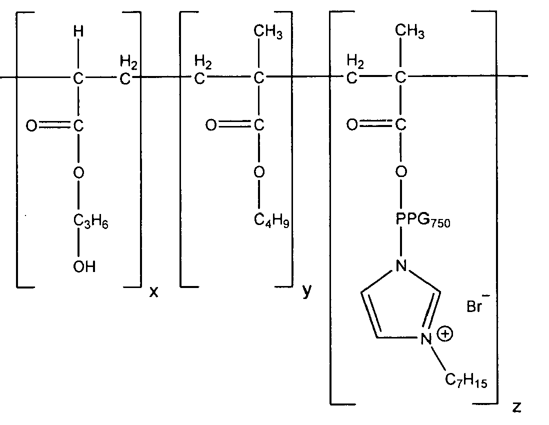 | x=2, y=1 and z=1 |
| 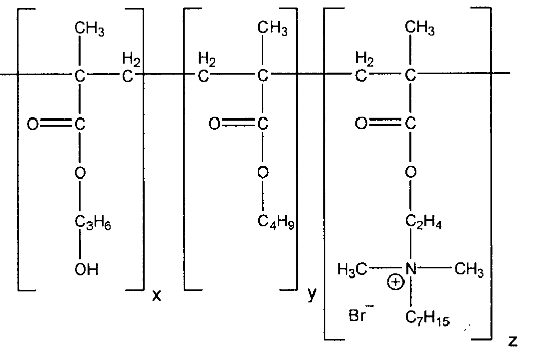 | X=1, y=2 and z=1 |
| 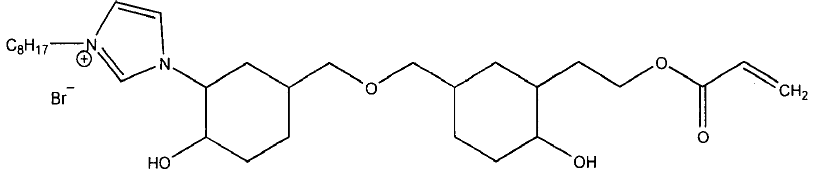 | |
| 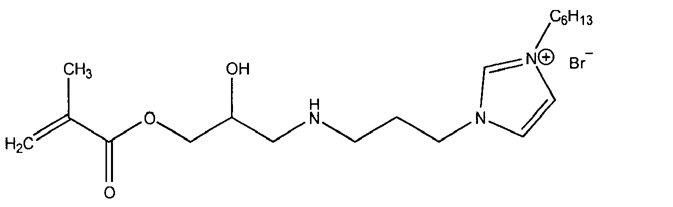 | |
| 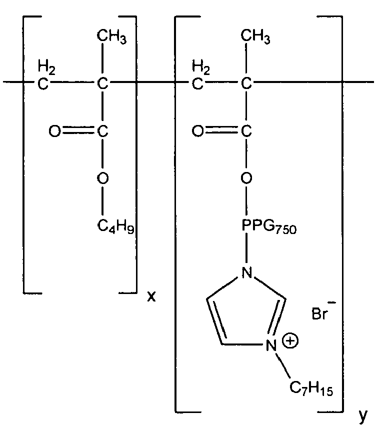 | x=2 and y=1 |
| | PPG750 = polypropylene |
| | glycol with a MW of 750 |
| 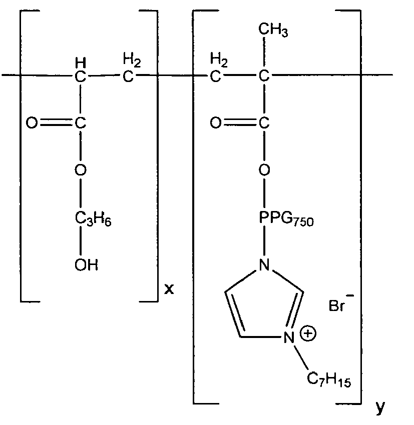 | x=1 and y=1 |
| | or |
| | x=4 and y=1 |
| | PPG750 = polypropylene glycol with a MW of 750 |
| 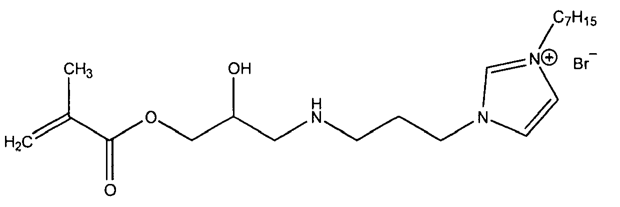 | |
| 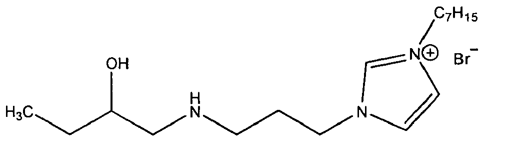 | |
| 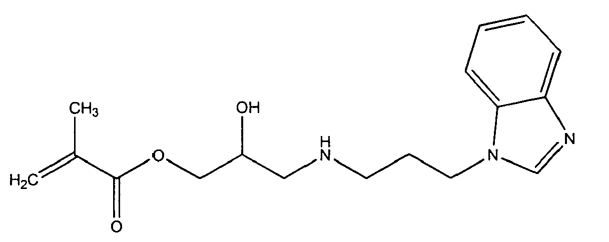 | |
| 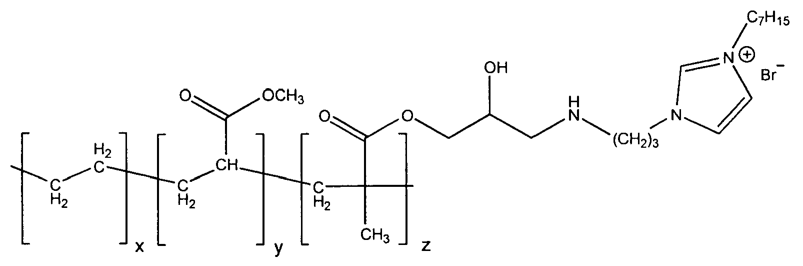 | X=5-15% |
| | y=70-90% |
| | z=5-15% |

Alternatively, the chemical compound to be deposited on the substrate is composed of at least two groups, wherein at least a first group is activated by the plasma species e.g. radicals such as N, O, O2-, HO2, OH, which improves and/or provides binding of the chemical compound to the surface of the material to be treated, and/or said activated group forms a polymer; and wherein at least a second group has antimicrobial activity, but shows essentially no or only a minimal activation by plasma, or, alternatively, of which the antimicrobial activity is introduced or enhanced by plasma. The advantage of using such chemical compounds is that the antimicrobial activity is not destroyed by plasma and/or the subsequent binding and/or polymerisation.

The antimicrobial active compound is advantageously combined with an agent that provides another functionality to the coating in order to obtain a multifunctional coating. Suitable agents include for instance colour pigments for colouration to withstand fading, UV agents to withstand fading and degradation in products exposed to significant UV light, agents to make the surface of the substrate hydrophilic, hydrophobic or oleophobic; agents to make the product flame retardant or flame resistant, agents to improve printability, agents to modify frictional properties, agents to promote adhesion, bioactive agents and/or agents to make the product anti-static.

The antimicrobial active compound may be combined with the other functional agent in a single compound, but it is also possible that the other functional agent is a separate compound that is applied together with the antimicrobial active compound on the surface of the substrate.

In a further aspect, the invention is directed to the use of a plasma treatment in the preparation of an antimicrobial coating for enhancing the antimicrobial properties of said coating.

The invention is further directed to a substrate obtainable by the process of the invention. As mentioned hereinabove, it was found that substrates with a polymer coating obtained by the assistance of plasma treatment in accordance with the invention not only have improved properties over a substrate with a polymer coating obtained by conventional wet processing techniques, but are also different in general structure, in the sense that much thinner coating layers are obtained. A substrate obtainable by the process of the invention can be advantageously used to enhance the functionality of existing products, or they can be further processed into or directly used as for instance products against biological and chemical hazards and for personal protection products for example for police and military personnel, a packaging material, filters, face masks, anti-allergic products, cleaning products, hygiene articles, sports and apparel textiles, footwear, leather and technical textiles. Technical textiles according to a definition published by the textile institute, is defined as textile material and products manufactured primarily for their technical performance and functional properties rather than their aesthetic or decorative characteristics.

| | | | | |
|---|---|---|---|---|
| **Table- 2** | | | | |

| **Construction Textile** | **Medical Textile** | **Industrial Articles** | **Mobile Textile** | **Protective Textile** |
|---|---|---|---|---|
| • Geo textile for under water and under ground construction | • Hygiene articles and Cosmetics | • Composite fabrics as alternative material | • Automobile interior design | • Defense textile |
| | | | • Transportation tarpaulin | • Technical goods |
| | • Hospital liners | | | |
| | | • Ape article and dressing | | |
| | | | • Aviation and space travel | • Heat, cold fire, chemical protection |
| • Textile for civil Engineering (reinforcing, roof and tents) | • Surgical articles and dressing | | | |
| | | • Filter fabrics and sieves | | |
| | | | • Tyre , V-belt and drive belts | |
| | | | | • Weather protection and sport clothing |
| | | • Polishing textiles (sanding, cleaning cloths) | | |
| | | | • Transport belt, conveyer belts | |
| • Ag ro covering | | • Roller covering Packing material Dyestuff carrier circuit board | • Seal, brake lining | |
| • Screening and Sound insulation | | | | |

Technical textile is the most intelligent use of the textile material technically used by the industries of non-conventional character in high tech and high performance application starting from auto motive engineering to building and personal protection. As can be seen in the above Table 2, the vast application includes advertising, agriculture, automobile, aviation, civil engineering, chemical, electrical industries, leather, medical, environmental, protection, etc.

The substrate obtainable by the process of the invention may thus also be used for example for interior design of automobile, ship, aviation and space travel, carpets, leather and textiles for business and home use such as for example seat covers, curtains towels and linen, textiles for medical purposes such as for example bandages, medical garment, bed linen and surgical drape, paper used for instance in building and construction and as a filling material for example for furniture and screens. Historical textile, leather and paper can be treated by the process of invention to achieve antimicrobial protection together with for example protection against UV radiation.

### Example

### EXAMPLE 1- General method for determining antibacterial activity

As the antibacterial activity depends on the nature of samples that are coated, treated samples and a clean (not treated) sample was always tested together. *Escherichia coli* ATCC 11775 was used for testing. At T=0 hours the total living bacteria, which was possible to clean from the material, were counted. After 24 hours of contact time at 37°C the surviving bacteria were counted again. With the numbers of bacteria at T=0 hours and T=24 hours, the antibacterial activity was calculated

### Method

### Pieces of cloth

Treated samples and one clean sample of cloth were used. The samples of cloth were divided into four equal pieces and every piece was placed in a sterile Petridish.

### Culture

*Escherichia coli* ATCC 11775 was cultured overnight at 37°C and 150 rpm in Brain Heart Infusion broth (BHI). 1 ml of overnight culture was added to 10 ml of BHI. This pre-culture was incubated for 2 hours at 37°C and 150 rpm.

### Contamination piece of cloth

All four pieces of cloth were contaminated with a 100 µl pre-culture. The drops were evenly spread over each piece of cloth with a spreader. Three pieces of cloth were incubated for 24 hours at 37°C while drying-up was prevented. One piece of cloth was prepared immediately (i.e. at T=0 hours). After the incubation, the surviving rate was determined. The pieces of cloth were placed in 5 ml physiological salt (FZ) or BHI. The tubes were vortexed for 2 minutes. From the FZ, a 10 fold dilution series was made in FZ.

### Plating

From each dilution 100µl was spread on a Tryptic Soy Agar plate (TSA) or Luria broth agar plate (LB). The plates were incubated for 18 hours at 37°C. After 18 hours the already formed colonies were counted. The plates were returned to the incubator for another 3 days at 37°C. After 3 days the colonies were counted again.

### Desired quantified results

The number of (surviving) bacteria was expressed as colony forming units (cfu). The antibacterial activity (R-value) was calculated as: log cfu T0 (control) - log cfu T24 (test material).

### EXAMPLE 2 - Antibacterial activity of a coating of the invention

Plasma assisted grafting of poly(ethylene-co-methylacrylate-co-glycidylmethacrylate modified with N-hexylated 1-(3-aminopropyl)-imidazole was used for the deposition of antimicrobial and hydrophilic coating on a cotton substrate.

Dipping of cotton into the chemical and subsequent drying yielded a destruction efficiency of *Escherichia coli* (R of 4.1). Dipping of cotton into the same chemical, but performed after plasma activation in nitrogen plasma and nitrogen-water vapour plasma resulted in an R value of 4.6 and 5.2, respectively. Plasma activation was done with the help of surface dielectric barrier discharge.

## Claims

1. Process for preparing a substrate with an antimicrobial coating comprising:
- providing a substrate;
- subjecting a surface of the substrate to a plasma environment; and
- reacting an antimicrobial active compound on the surface of the substrate.

2. Process according to claim 1, wherein at least part of the antimicrobial active compound is reacted on the surface of the substrate simultaneously with subjecting the surface of the substrate to the plasma environment.

3. Process according to claim 1, wherein at least part of the antimicrobial active compound is reacted on the surface of the substrate after the surface of the substrate has been subjected to the plasma environment.

4. Process according to claim 1, wherein at least part of the antimicrobial active compound is applied on the surface of the substrate before the surface of the substrate having the applied antimicrobial active compound is subjected to the plasma environment.

5. Process according to any of claims 2 to 4, wherein the antimicrobial active compound is polymerised.

6. Process according to any one of the preceding claims, wherein the antimicrobial active compound comprises a quaternary ammonium moiety.

7. Process according to any one of the preceding claims, wherein the antimicrobial active compound comprises an optionally substituted aromatic heterocycle with at least one nitrogen atom.

8. Process according to claim 7, wherein the aromatic heterocycle with at least one nitrogen atom is selected from the group consisting of imidazoles, triazoles, tetrazoles, pyridines, pyrroles, pyrimidines, pyridazine, pyrazine, purine or a derivative thereof.

9. Process according to any one of the preceding claims, wherein the antimicrobial active compound is selected from the compounds listed in Table 1.

10. Process according to any one of the preceding claims, wherein the antimicrobial active compound comprises a reactive group selected from the group consisting of a (meth)acrylate or a derivative thereof, a hydroxyl or an amino group.

11. Process according to claim 10, wherein the reactive group is bound through an isocyanate, glycidyl, an alcohol, an epoxide, a vinyl or an allyl moiety.

12. Process according to any one of the preceding claims, wherein the plasma environment is applied by surface dielectric barrier discharge or coplanar surface dielectric barrier discharge.

13. Process according to any one of claims 1-11, wherein the plasma environment is applied by a plasma jet or microwave plasma source.

14. Process according to any one of claims 1-11, wherein the plasma environment is applied by corona discharge, dielectric barrier discharge, atmospheric pressure glow discharge, volume filamentary dielectric barrier discharge, volume glow dielectric barrier discharge and micro-hollow cathode discharge.

15. Process according to any one of the preceding claims, wherein the coating is a multifunctional coating.

16. Process according to claim 15, wherein the antimicrobial compound is combined with one or more agents that provides at least one functionality other than antimicrobial to the coating.

17. Process according to claim 15 or 16, wherein the multifunctional coating has at least one functionality selected from the group consisting of hydrophilic, hydrophobic, oleophobic, flame retardant or flame resistant, UV protection, bioactive and anti-static.

18. Use of a plasma treatment in the preparation of an antimicrobial coating for enhancing the antimicrobial properties of said coating.

19. Use of a reactive antimicrobial active compound with general formula (I) 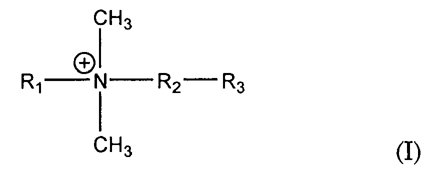 in a process according to any one of claims 1-16, wherein
R₁ is a C₃₋₁₂ alkyl group, preferably a C₅₋₉ alkyl group, more preferably a C₇ or C₈ alkyl group;
R₂ is an optionally substituted C₁₋₆ chain, preferably a C₂₋₄ chain; and
R₃ is chosen from the group consisting of an acrylate, a diisocyanate, a diol, an ethylene, and a styrene.

20. Use of a reactive antimicrobial active compound with general formula (II) 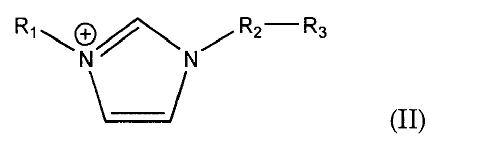 in a process according to any one of claims 1-16, wherein
R₁ is a C₃₋₁₂ alkyl group, preferably a C₅₋₉ alkyl group, more preferably a C₇ or C₈ alkyl group;
R₂ is an optionally substituted C₁₋₆ chain, preferably a C₂₋₄ chain; and
R₃ is chosen from the group consisting of an acrylate, a diisocyanate, a diol, an ethylene, and a styrene.

21. Use of a reactive antimicrobial active compound selected from Table 1 in the description in a process according to any one of claims 1-17.

22. Substrate with an antimicrobial coating obtainable by a process according to any one of claims 1-17.

23. Substrate according to claim 22, which substrate is chosen from the group consisting of textile, nanofibre non-woven, felt, yarn, fibre, paper, foil, membranes, leather, polymers, wood and ceramics.

24. Substrate according to claim 22 or 23 in the form of a filter, anti-allergic product, hygiene article or textile for medical purposes, such as bandages, medical garment, bed linen and surgical drape.

25. Substrate according to claim 22 or 23 in the form of sports textile, apparel textile or footwear.

26. Substrate according to claim 22 or 23 in the form of leather and technical textile, such as textile for interior design of automobile, ship, aviation and space travel.

27. Substrate according to claim 22 or 23 in the form of a carpet, or leather or textiles for business and home use, such as seat covers, curtains towels and linen.

28. Substrate according to claim 22 or 23 in the form of a packaging material.

29. Use of a substrate according to claim 22 or 23 in building and/or construction, or as filling material.

30. Substrate according to claim 22 or 23 in the form of historical patrimony such as for example historical textile, leather, parchment and manuscripts.

31. Use of a substrate according to claim 22 or 23 for protection against biological and/or chemical hazards and/or for personal protection products, for example for police and military personnel.
